# EUROPEAN PATENT SPECIFICATION

(11) **EP 2 522 650 B1**
(45) Date of publication and mention of the grant of the patent: **09.03.2016**
(21) Application number: 11166000.7
(22) Date of filing: 13.05.2011
(51) Int. Cl.: C07C 29/86, C07C 29/149, C07C 51/09, C07C 53/00, C07C 53/126, C07C 29/80

(54) **Process for purifying crude fatty alcohols**
Verfahren zur Reinigung von rohen Fettalkoholen
Procédé de purification d'alcools gras bruts

(43) Date of publication of application: 14.11.2012
(73) Proprietor: Cognis IP Management GmbH, 40589 Düsseldorf (DE)
(72) Inventor: Josten, Horst, 40593 Düsseldorf (DE); Dimitrova, Pepa, 40591 Düsseldorf (DE); Tran Anh, Truc, 40595 Düsseldorf (DE)

(56) References cited:
- WO-A1-90/08123
- WO-A2-2009/100902
- GB-A- 825 359
- GB-A- 1 173 217
- KREUTZER U R: "MANUFACTURE OF FATTY ALCOHOLS BASED ON NATURAL FATS AND OILS", JOURNAL OF THE AMERICAN OIL CHEMISTS' SOCIETY. (JAOCS) ED.2, SPRINGER, DE, vol. 61, no. 2, 1 February 1984 (1984-02-01), pages 343-348, XP000673352, ISSN: 0003-021X, DOI: 10.1007/BF02678792

## Description

### Field of invention

The present invention belongs to the area of fatty alcohol production and refers to an improved method for purifying streams of fatty alcohols coming of the respective hydrogenation of fatty acid methyl esters.

### State of the art

Fatty alcohols represent important raw materials for various types of consumer products, as for example cosmetics and detergents. Typically, they are obtained from vegetable oils for example by transesterification with methanol to form methyl esters and subsequent hydrogenation. The methyl esters for example from coconut or palm kernel oil are typically split into fractions C₆₋₁₀, C₁₂₋₁₄ and C₁₆₋₁₈ by distillation in fractionation columns under vacuum. The different fractions are then hydrogenated separately.

In the hydrogenation step the methyl esters are converted to fatty alcohols and methanol. Due to side reactions additional by-products are formed such as for example water, hydrocarbons and wax esters. Furthermore, since the hydrogenation conversion is not 100 % complete, residual methyl esters are remaining in the crude fatty alcohol fractions. Obviously, it is desirous to remove these unwanted by-products to a level which is acceptable in terms of the later conversion or application.

Therefore, it represents state of the art to remove methanol and water from the crude fatty alcohols by stripping in a simple flash distillation unit. In order to separate also other low boilers like hydrocarbons and remaining traces of methanol and water, an additional fractionation in a top cut column is typically applied. For removal of high boilers such as wax esters the fatty alcohols are further fractionated in an overhead distillation column. Alternatively the fatty alcohols can be purified in one column with side stream withdrawal. As a matter of fact, these standard operations require a lot of energy and technical equipment. Also, the waste material, although having a high content of organics, does not have any application and is typically used as an - quite expensive - fuel for generating steam and energy.

This prior art is disclosed in - KREUTZER U R: "MANUFACTURE OF FATTY ALCOHOLS BASED ON NATURAL FATS AND OILS", JOURNAL OF THE AMERICAN OIL CHEMISTS' SOCIETY. (JAOCS) ED.2, SPRINGER, DE, vol. 61, no. 2, 1 February 1984 (1984-02-01), pages 343-348, XP000673352, ISSN: 0003-021X, DOI: 10.1007/BF02678792, which forms the preamble of claim 1.

GB 825 359 A discloses the purification of fatty alcohols obtained from an oxo process. This prior art discloses a process for the purification of ester containing raw alcohols having 6-19 carbon atoms by treatment with an alcoholic alkaline solution followed by washing with water to separate the fatty alcohols. An oily layer containing the fatty alcohol and an aqueous layer is obtained.

GB 1 173 217 A discloses the purification of fatty alcohols obtained from a sodium reduction process. This prior art discloses a process for producing fatty alcohols from fats and oils comprising the steps of esterifying the fats or oils with butanol in order to form the corresponding butyl esters, reducing the resulting butyl esters with sodium to obtain a mixture comprising sodium butylate, glycerol, aliphatic alcohols and a minor quantity of soaps, heating said mixture with water to hydrolyze said sodium butylate to sodium hydroxide and butanol and washing said hydrolyzed mixture to eliminate the sodium hydroxide, soaps and glycerol therefrom and obtain an aqueous butanolic solution of aliphatic alcohols.

It has therefore be the object of the present invention to develop an improved process for the purification of fatty alcohols, in particular for removing esters from the hydrogenated fractions, which is more efficient, easier to handle and of course cheaper. Simultaneously the process should provide the unwanted by-products in a quality, which can be sold as a valuable product.

### Description of the invention

Object of the present invention is a process for simultaneously purifying fatty alcohols and obtaining a fraction of free fatty acids,
whereas
(a) the fatty alcohols which are subjected to purification are technical grade fatty alcohols which are obtained from renewable oils and fats, like e. g. palm oil, palm kernel oil, coconut oil, olive oil, soy oil, rape seed oil, tallow and fish oil
(b) the fatty alcohols which are subjected to purification either comprise
   (i) 12 to 18 carbon atoms and are obtained as the distillate from the hydrogenation of the respective alkyl esters, or
   (ii) 6 to 36 carbon atoms and are obtained as the distillation bottom from the hydrogenation of the respective alkyl esters
(c) crude fatty alcohols comprising up to 70 % b.w. alkyl esters and/or wax esters are treated with alkaline bases in an amount sufficient to essentially convert said esters into alkaline soaps,
which is characterised in that
(d) said alkaline soaps are separated from said fatty alcohols by extraction to produce a purified fraction of fatty alcohols and an aqueous soap phase, and
(e) said soap phase is treated with strong mineral acids in order to release the fatty acids from the soaps.

Surprisingly, it has been observed that even huge parts of methyl esters and/or wax esters can be removed from fatty alcohols by transesterification using alkaline bases and forming alkali soaps according to the following equations:

R-C00-R'+ NaOH→R-COO-Na + R'-OH

RCOO-CH₃ + NaOH → R-COO-Na + CH₃OH

According to the present invention it is possible to reduce the saponification number to a value of at most 0.5, representing a typical specification for fatty alcohols. Compared to what is known from the state of the art and indeed is found in the market, the process is easier to conduct and also more advantageous in terms of energy consumption and the production of a separate phase of free fatty acids, which can be sold as a valuable product rather than to be treated as none-value waste material. In particular, the advantages are
- Increased product yield, since the wax esters are converted to fatty alcohols and alkali soaps. The yield loss is only approximately 50 % (w/w) of the wax ester concentration. In the state of the art process the wax esters are completely lost to the distillation residue.
- No loss of fatty alcohol via distillation.
- Energy reduction due to elimination of the overhead distillation step.
- Savings with regard of high pressure steam, cooling water and motive steam for the vacuum unit.
- The colour quality of the fatty alcohol is not affected, since the transesterification is performed at low temperature.

### Fatty alcohols

Preferably, the fatty alcohols which are subjected to purification are following general formula (I)

R¹OH (I)

in which R¹ stands for a saturated or unsaturated, linear or branched and optionally hydroxyl-substituted hydrocarbon residue with 6 to 36 carbon atoms and 0 or 1 to 5 double bonds. Typical examples are capryl alcohol, caprinyl alcohol, lauryl alcohol, myristyl alcohol, cetyl alcohol, palmoleyl alcohol, stearyl alcohol, cetearyl alcohol, oleyl alcohol, linoly alcohol, linolenyl alcohol, elaidyl alcohol, 12-hydroxy stearyl alcohol, ricinolyl alcohol, gadoleyl alcohol, erucyl alcohol, behenyl alcohol, their mixtures and the technical grade fatty alcohols which can be obtained from renewable oils and fats, like e. g. palm oil, palm kernel oil, coconut oil, olive oil, soy oil, rape seed oil, tallow and fish oil.

The fatty alcohols which are subjected to purification either comprise
(i) 12 to 18 carbon atoms and are obtained as the distillate from the hydrogenation of the respective alkyl esters, or
(ii) 6 to 36 carbon atoms and are obtained as the distillation bottom from the hydrogenation of the respective alkyl esters.

Typically the crude fatty alcohols comprise 1 to 60 % b.w. alkyl esters and/or 5 to 40 % b.w. wax esters.

### Purification of fatty alcohol distillates

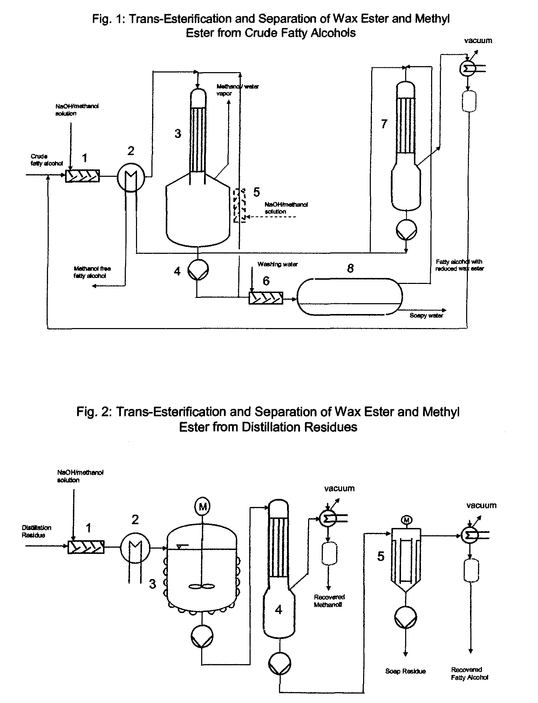

The process is illustrated by Figure 1. In a first embodiment, the crude fatty alcohols from the hydrogenation step are mixed with alcoholic alkaline bases, preferably NaOH or KOH in methanol in mixing equipments, preferably a static mixer (1), the mixture is preheated via economizer (2) and routed to a first evaporator, preferably a falling film evaporator (3). The reaction of alkyl and/or wax esters with the bases occurs mainly during the heating in economizer and evaporator. Preferably, the evaporator is designed with a large bottom vessel in order to provide the required reaction time of approximately 10 to 30 min. Homogenizing and mixing of the reaction mixture is typically done by a recirculation pump (4). Mixing efficiency can be controlled by adjusting the recirculation rate. As an alternative the alcoholic alkaline solution can be added to the crude fatty alcohol in a static mixer (5), which is installed in the recirculation line. The temperature in the receiving vessel is adjusted to about 80 to about 120, and preferably about 90 to about 110°C by steam control to the evaporator. The methanol concentration in the bottom product at these temperatures is typically about 5 to about 10 % b.w., which is necessary for a good dissolution of alkaline base in the fatty alcohol.

It is also preferred to take off a part of the reaction mixture from the recirculation loop, mixed with water at about 90 to about 120 °C preferably in static mixer (6) and fed to a gravity separator (8), where the mixture splits into a light fatty alcohol phase reduced in wax ester content and a heavy water phase containing the alkaline soaps. The temperature in the separation vessel is preferably about 80 to about 120 °C to enable a good phase separation. The separator is operated typically at about 1 to about 3 barg in order to avoid the evaporation of methanol and water. Depending on the required minimum alkali concentration in the fatty alcohol phase an additional washing step can be performed counter-currently in one or two steps.

Subsequently, the fatty alcohol is taken off from the phase separator as the light phase and is routed to a second evaporator, also preferably a falling film evaporator (7), where the alcohols, e.g. methanol is removed by distillation. The water phase is routed to a soap splitting step for recovery of the fatty acid as described above.

### Purification of fatty alcohol residues

Basically, a second embodiment of the present invention refers to a process for the purification of high boiling fractions, according to which crude fatty alcohol residues are mixed with alcoholic alkaline bases and subjected to transesterification in a stirred vessel to produce a intermediate transesterification product that is subjected to a two-step distillation to produce a purified stream of fatty alcohols. The residues from the distillation steps are subjected to a treatment with strong mineral acids to obtain the free fatty acids.

Therefore, an alternative procedure of the invention is saponifying distillation residues from the current fatty alcohol distillations with alkaline bases in methanol or other alcohols in order to split the wax esters contained in the fatty alcohols and distilling the mixture to recover fatty alcohols as set out in Figure 2. Typically, the distillation residues contain wax esters in the range of about 30 to about 70 % b.w., the remainder being mainly fatty alcohols. During saponification with alkaline bases like NaOH or KOH the wax esters are transformed into fatty acid soaps and free fatty alcohols. The reaction mixture should then be supplied to a distillation, preferably a two-step distillation for example in a wiped film evaporator, where the free fatty alcohols are recovered almost quantitatively from the mixture. The loss of fatty alcohols in the soap residue from the evaporator can be kept in the range of about 5 to about 30 % b.w.

Preferably, the distillation residue and alcoholic alkaline bases are continuously fed via a static mixer (1) and pre-heater (2) to a stirred vessel (3), where the transesterification is applied at a temperature of about 120 °C and a residence time of about 30 min. The reaction mixture is then first routed to a methanol stripper (4) - for example a falling film evaporator - where the mixture is distilled at about 500 mbar to atmospheric pressure and finally to a second evaporator, also preferably a wiped film evaporator (5), where the mixture is distilled at a vacuum of about 1 to about 10 mbar and temperatures up to about 250 °C. The recovered fatty alcohols are condensed and the alkaline soaps are discharged from the evaporator as residue.

### Soap splitting

The liberation of the free fatty acids from their alkaline soaps can be conducted according to the state of the art, for example by treating the aqueous solution with strong mineral acids like sulphuric acid or hydrochloric acid. Basically, it is also possible to use any other organic acid that is strong enough to replace the fatty acids in their salts. Once the splitting has taken place, the crude fatty acids are washed with water and separated from the aqueous phase by gravity settling.

### Examples

### Example 1

100 g of C₁₂/C₁₄ fatty alcohol with saponification number 0.88 and wax ester content of 0.51 % (w/w) were mixed with 14 g 0.1 mol/l NaOH in methanol and heated in a flask for 60 min at 85°C under reflux. Methanol was then removed by distillation in a rotary evaporator. After that 20 g of deionised water were added and the product was washed in the rotavapor for 30 min at 80°C. The mixture was then decanted in a separating funnel and the organic phase dried in the rotary evaporator. The resulting saponification number of the organic phase was 0.38 and wax ester content 0.26 % (w/w). Measured sodium content in the organic phase was 150 ppm and 1,700 ppm in the aqueous phase.

### Example 2

100 g C₁₂/C₁₄ fatty alcohol with a saponification number of 0.88 and wax ester content of 0.51 % (w/w) were mixed with 14 g 0.1 mol/l KOH in methanol and heated in a flask for 60 min at 85°C under reflux. Methanol was then removed with a rotary evaporator. Afterwards 20 g deionised water was added and the product was washed for 30 min at 80°C in the rotary evaporator. The mixture was then decanted in a separating funnel. After phase separation the organic phase was dried in the rotary evaporator. Resulting saponification number was 0.38 and wax ester content 0.25 % (w/w). Measured potassium content was 330 ppm in the organic phase and 2,800 ppm in the aqueous phase.

### Example 3

100 g of a distillation residue containing 6 % b.w. methyl ester, 34 % b.w. wax ester and 60 % b.w. free fatty alcohol was mixed with 120 g 0.1 mol/l NaOH in methanol and reacted for 30 min at 120°C in an autoclave. Subsequently, methanol was distilled from the mixture in a rotary evaporator at 130°C and atmospheric pressure. The resulting mixture had a residual methanol content of 1.1 % (w/w). The mixture was then fed to a wiped film evaporator at 1 mbar and 235°C. 71 g distillate was achieved containing 1.6 % methanol, the rest free fatty alcohols. The remaining 35 g of residue still contained 26.2 % (w/w) free fatty alcohol.

## Claims

1. Process for simultaneously purifying fatty alcohols and obtaining a fraction of free fatty acids, whereas
(a) the fatty alcohols which are subjected to purification are technical grade fatty alcohols which are obtained from renewable oils and fats, like e. g. palm oil, palm kernel oil, coconut oil, olive oil, soy oil, rape seed oil, tallow and fish oil
(b) the fatty alcohols which are subjected to purification either comprise
(i) 12 to 18 carbon atoms and are obtained as the distillate from the hydrogenation of the respective alkyl esters, or
(ii) 6 to 36 carbon atoms and are obtained as the distillation bottom from the hydrogenation of the respective alkyl esters
(c) crude fatty alcohols comprising up to 70 % b.w. alkyl esters and/or wax esters are treated with alkaline bases in an amount sufficient to essentially convert said esters into alkaline soaps,
**characterised in that**
(d) said alkaline soaps are separated from said fatty alcohols by extraction to produce a purified fraction of fatty alcohols and an aqueous soap phase, and
(e) said soap phase is treated with strong mineral acids in order to release the fatty acids from the soaps.

2. Process according to Claim 1, **characterised in that** fatty alcohols are subjected to purification following general formula (I)
R¹OH (I)
in which R¹ stands for a saturated or unsaturated, linear or branched and optionally hydroxyl-substituted hydrocarbon residue with 6 to 36 carbon atoms and o or 1 to 5 double bonds.

3. Process according to Claims 1 and/or 2, **characterised in that** said crude fatty alcohols comprise 1 to 60 % b.w. alkyl esters and/or 5 to 40 % b.w. wax esters.

4. Process according to any of the preceding Claims 1 to 3, **characterised in that** the purification of the crude fatty alcohols according to step (a) leads to a fraction of purified fatty alcohols showing a saponification number of at most 0.5.

5. Process according to Claim 1, **characterised in that** said crude fatty alcohol distillates are mixed with alcoholic alkaline bases and subjected to transesterification in an evaporator.

6. Process according to Claim 5, **characterised in that** said evaporator is a falling-film evaporator.

7. Process according to Claims 5 and/or 6, **characterised in that** said evaporator is operated at a temperature of from 80 to 170°C.

8. Process according to any of the preceding Claims 5 to 7, **characterised in that** a first part of the reaction product leaving the evaporator is recycled and fed to the top of the evaporator.

9. Process according to any of the preceding Claims 5 to 8, **characterised in that** a second part of the reaction product leaving the evaporator is mixed with water and fed to a gravity separator, where the mixture is separated into a first light fatty alcohol phase and a heavy water phase containing the alkaline soaps.

10. Process according to any of the preceding Claims 5 to 9, **characterised in that** said light fatty alcohol phase is subjected to a distillation in a second evaporator in order to remove remaining alcohols, while said heavy water phase is subjected to a treatment with strong mineral acids to obtain the free fatty acids.

11. Process according to Claim 1, **characterised in that** said crude fatty alcohol residues are mixed with alcoholic alkaline bases and subjected to transesterification in a stirred vessel to produce an intermediate transesterification product that is subjected to a two-step distillation to finally produce a purified stream of fatty alcohols.

12. Process according to Claims 1 and/or 11, **characterised in that** the residues the distillation steps, containing the fatty acid soaps, is subjected to a treatment with strong mineral acids to obtain the free fatty acids.

13. Process according to any of the preceding Claims 1 to 12, **characterised in that** mineral acids are applied selected from the group consisting of sulphuric acid and hydrochloric acid.

## Patentansprüche

1. Verfahren zum gleichzeitigen Reinigen von Fettalkoholen und Erhalten einer Fraktion freier Fettsäuren, wobei
(a) es sich bei den Fettalkoholen, die der Reinigung unterworfen werden, um Fettalkohole technischer Qualität, die aus erneuerbaren Ölen und Fetten, wie z.B. Palmöl, Palmkernöl, Kokosnussöl, Olivenöl, Sojaöl, Rapsöl, Talg und Fischöl erhalten werden, handelt,
(b) die Fettalkohole, die der Reinigung unterworfen werden, entweder
(i) 12 bis 18 Kohlenstoffatomse umfassen und als Destillat aus der Hydrierung der jeweiligen Alkylester erhalten werden oder
(ii) 6 bis 36 Kohlenstoffatome umfassen und als Destillationssumpf aus der Hydrierung der jeweiligen Alkylester erhalten werden,
(c) rohe Fettalkohole, die bis zu 70 Gew.-% Alkylester und/oder Wachsester umfassen, mit alkalischen Basen in einer zur weitgehenden Umwandlung der Ester in alkalische Seifen ausreichenden Menge behandelt werden,
**dadurch gekennzeichnet, dass**
(d) die alkalischen Seifen durch Extraktion von den Fettalkoholen getrennt werden, was eine gereinigte Fraktion von Fettalkoholen und eine wässrige Seifenphase ergibt, und
(e) die Seifenphase zur Freisetzung der Fettsäuren aus den Seifen mit starken Mineralsäuren behandelt wird.

2. Verfahren nach Anspruch 1, **dadurch gekennzeichnet, dass** die Fettalkohole, die der Reinigung unterworfen werden, der allgemeinen Formel (I) entsprechen:
R¹OH (I),
in der R¹ für einen gesättigten oder ungesättigten, linearen oder verzweigten und gegebenenfalls hydroxylsubstituierten Kohlenwasserstoffrest mit 6 bis 36 Kohlenstoffatomen und 0 oder 1 bis 5 Doppelbindungen steht.

3. Verfahren nach Anspruch 1 und/oder 2, **dadurch gekennzeichnet, dass** die rohen Fettalkohole 1 bis 60 Gew.-% Alkylester und/oder 5 bis 40 Gew.-% Wachsester umfassen.

4. Verfahren nach einem der vorhergehenden Ansprüche 1 bis 3, **dadurch gekennzeichnet, dass** die Reinigung der rohen Fettalkohole gemäß Schritt (a) zu einer Fraktion von gereinigten Fettalkoholen mit einer Verseifungszahl von höchstens 0,5 führt.

5. Verfahren nach Anspruch 1, **dadurch gekennzeichnet, dass** die rohen Fettalkoholdestillate mit alkoholischen alkalischen Basen gemischt und einer Umesterung in einem Verdampfer unterworfen werden.

6. Verfahren nach Anspruch 5, **dadurch gekennzeichnet, dass** es sich bei dem Verdampfer um einen Fallfilmverdampfer handelt.

7. Verfahren nach Anspruch 5 und/oder 6, **dadurch gekennzeichnet, dass** der Verdampfer bei einer Temperatur von 80 bis 170°C betrieben wird.

8. Verfahren nach einem der vorhergehenden Ansprüche 5 bis 7, **dadurch gekennzeichnet, dass** ein erster Teil des aus dem Verdampfer austretenden Reaktionsprodukts rezykliert und dem Kopf des Verdampfers zugeführt wird.

9. Verfahren nach einem der vorhergehenden Ansprüche 5 bis 8, **dadurch gekennzeichnet, dass** ein zweiter Teil des aus dem Verdampfer austretenden Reaktionsprodukts mit Wasser gemischt und einem Schwerkraftabscheider zugeführt wird, in dem die Mischung in eine erste leichte Fettalkoholphase und eine schwere Wasserphase, die die alkalischen Seifen enthält, getrennt wird.

10. Verfahren nach einem der vorhergehenden Ansprüche 5 bis 9, **dadurch gekennzeichnet, dass** die leichte Fettalkoholphase zur Abtrennung verbliebener Alkohole einer Destillation in einem zweiten Verdampfer unterworfen wird, während die schwere Wasserphase einer Behandlung mit starken Mineralsäuren zum Erhalt der freien Fettsäuren unterworfen wird.

11. Verfahren nach Anspruch, 1, **dadurch gekennzeichnet, dass** die rohen Fettalkoholrückstände mit alkoholischen alkalischen Basen gemischt werden und einer Umesterung in einem Rührkessel unterworfen werden, was ein intermediäres Umesterungsprodukt ergibt, welches einer 2-stufigen Destillation unterworfen wird, was schließlich einen gereinigten Strom von Fettalkoholen ergibt.

12. Verfahren nach Anspruch 1 und/oder 11, **dadurch gekennzeichnet, dass** die Rückstände der Destillationsschritte, die die Fettsäureseifen enthalten, einer Behandlung mit starken Mineralsäuren zum Erhalt der freien Fettsäuren unterworfen werden.

13. Verfahren nach einem der vorhergehenden Ansprüche 1 bis 12, **dadurch gekennzeichnet, dass** Mineralsäuren aus der Gruppe bestehend aus Schwefelsäure und Salzsäure eingesetzt werden.

## Revendications

1. Procédé destiné à purifier simultanément des alcools gras et obtenir une fraction d'acides gras libres, dans lequel
(a) les alcools gras étant soumis à une purification sont des alcools gras de qualité technique qui sont obtenus à partir d'huiles et de matières grasses renouvelables, telles que par exemple l'huile de palme, l'huile de palmiste, l'huile de coprah, l'huile d'olive, l'huile de soja, l'huile de colza, le suif et l'huile de poisson,
(b) les alcools gras étant soumis à une purification comprennent
(i) de 12 à 18 atomes de carbone et sont obtenus sous forme de distillat à partir de l'hydrogénation des esters d'alkyle respectifs, ou bien
(ii) de 6 à 36 atomes de carbone et sont obtenus sous forme de pied de distillation à partir de l'hydrogénation des esters d'alkyle respectifs,
(c) les alcools gras bruts comprenant jusqu'à 70% en poids d'esters d'alkyle et/ou d'esters de cire sont traités par des bases alcalines selon une quantité suffisante afin d'essentiellement convertir lesdits esters en savons alcalins,
**caractérisé en ce que**
(d) lesdits savons alcalins sont séparés desdits alcools gras par extraction afin de produire une fraction purifiée d'alcools gras et une phase de savon aqueuse, et
(e) ladite phase de savon est traitée par des acides minéraux forts afin de libérer les acides gras des savons.

2. Procédé selon la revendication 1, **caractérisé en ce que** les alcools gras sont soumis à une purification suivant la formule générale (I)
R¹OH (I)
où R¹ représente un reste hydrocarboné saturé ou insaturé, linéaire ou ramifié et éventuellement substitué par hydroxyle ayant de 6 à 36 atomes de carbone et 0 ou de 1 à 5 doubles liaisons.

3. Procédé selon les revendications 1 et/ou 2, **caractérisé en ce que** lesdits alcools gras bruts comprennent de 1 à 60% en poids d'esters d'alkyle et/ou de 5 à 40% en poids d'esters de cire.

4. Procédé selon l'une quelconque des revendications précédentes 1 à 3, **caractérisé en ce que** la purification des alcools gras bruts selon l'étape (a) conduit à une fraction d'alcools gras purifiés présentant un indice de saponification d'au plus 0,5.

5. Procédé selon la revendication 1, **caractérisé en ce que** lesdits distillats d'alcools gras bruts sont mélangés avec des bases alcalines alcooliques et soumis à une transestérification dans un évaporateur.

6. Procédé selon la revendication 5, **caractérisé en ce que** ledit évaporateur est un évaporateur à couches minces.

7. Procédé selon les revendications 5 et/ou 6, **caractérisé en ce qu'**on fait fonctionner ledit évaporateur à une température allant de 80 à 170°C.

8. Procédé selon l'une quelconque des revendications précédentes 5 à 7, **caractérisé en ce qu'**une première partie du produit de réaction quittant l'évaporateur est recyclée et alimentée dans la partie supérieure de l'évaporateur.

9. Procédé selon l'une quelconque des revendications précédentes 5 à 8, **caractérisé en ce qu'**une deuxième partie du produit de réaction quittant l'évaporateur est mélangée avec de l'eau et alimentée vers un séparateur par gravité, où le mélange est séparé en une première phase légère d'alcool gras et une phase aqueuse lourde contenant les savons alcalins.

10. Procédé selon l'une quelconque des revendications précédentes 5 à 9, **caractérisé en ce que** ladite phase légère d'alcool gras est soumise à une distillation dans un deuxième évaporateur afin d'éliminer les alcools restants, tandis que ladite phase aqueuse lourde est soumise à un traitement par des acides minéraux forts afin d'obtenir les acides gras libres.

11. Procédé selon la revendication 1, **caractérisé en ce que** lesdits résidus d'alcools gras bruts sont mélangés avec des bases alcalines alcooliques et soumis à une transestérification dans un récipient sous agitation afin de produire un produit de transestérification intermédiaire qui est soumis à une distillation en deux étapes afin de produire finalement un courant purifié d'alcools gras.

12. Procédé selon les revendications 1 et/ou 11, **caractérisé en ce que** les résidus des étapes de distillation, contenant les savons d'acides gras, sont soumis à un traitement par des acides minéraux forts afin d'obtenir les acides gras libres.

13. Procédé selon l'une quelconque des revendications précédentes 1 à 12, **caractérisé en ce que** des acides minéraux sont appliqués, lesquels acides sont choisis dans le groupe constitué par l'acide sulfurique et l'acide chlorhydrique.
